# FASCICULE DE BREVET EUROPEEN

(11) **EP 0 580 517 B1**
(45) Date de publication et mention de la délivrance du brevet: **21.03.2001**
(21) Numéro de dépôt: 93401912.6
(22) Date de dépôt: 23.07.1993
(51) Int. Cl.: A61M 16/10

(54) **Equipement et procédé de fourniture de doses d'au moins un gaz aux voies respiratoires d'un utilisateur**
Ausrüstung und Verfahren für das Zuführen von mindestens einem Gas in die Atemwege eines Benutzers
Equipment and process for supplying at least one gas into the airways of a user

(30) Priorité: 23.07.1992 FR 9209075
(43) Date de publication de la demande: 26.01.1994
(73) Titulaire: AIR LIQUIDE SANTE (INTERNATIONAL), 75007 Paris (FR)
(72) Inventeur: Willemot, Jean-Marie, F-92330 Sceaux (FR); Desforges, Daniel, F-95640 Santeuil (FR)
(74) Mandataire: Le Moenner, Gabriel

(56) Documents cités:
- EP-A- 0 188 071
- EP-A- 0 483 556
- WO-A-90/14121
- WO-A-91/06336
- US-A- 4 279 250
- US-A- 4 951 661

## Description

La présente invention concerne les équipements de fourniture aux voies respiratoires d'un utilisateur de doses d'au moins un gaz contenant des particules d'au moins un produit actif.

L'assistance respiratoire de patients ainsi que les traitements au moyen d'aérosols prennent une place de plus en plus importante dans la pathologie pulmonaire. Les équipements actuellement disponibles sont généralement spécifiques de certains traitements, n'offrent de ce fait qu'une piètre souplesse d'adaptation pour des utilisations diversifiées et ne garantissent pas un niveau de sécurité suffisant en l'absence d'un praticien, notamment en pédiatrie.

Le document EP-A-0188071 décrit un appareil pour administrer de l'air enrichi en oxygène à un patient en synchronisme avec les phases respiratoires du patient. Cet appareil comporte un régulateur permettant de contrôler la durée de l'administration d'oxygène dans le flux d'air pendant chaque inhalation. La durée d'administration est déterminée à partir de la moyenne des durées d'inhalation des phases inspiratoires précédentes et d'un rapport donné au régulateur.

En outre, le document US-A-4,951,661 décrit une valve-adaptateur insérée à l'intérieur d'un connecteur en T destinée à permettre la connection ou la déconnection rapide d'un nébuliseur à un appareil de ventilation artificielle d'un utilisateur. Cette valve-adaptateur permet de prévenir les contaminations bactériennes ou les fuites de fluide.

La présente invention, caractérisée dans les revendications, a pour objet de proposer un équipement simple, fiable, de faibles coûts de production et d'utilisation, offrant une grande souplesse d'adaptation et un niveau de sécurité élevé, tout en libérant l'utilisateur des contraintes de comptage et permettant un suivi de l'observance de prescription.

Pour ce faire, selon une caractéristique de l'invention, le dispositif de commande comprend, en série, dans un circuit de gaz entre une source de gaz et les voies respiratoires, une vanne de distribution de gaz et un nébuliseur, la vanne étant pilotée par un dispositif de commande comprenant un capteur sensible aux phases d'inspiration de l'utilisateur et des moyens programmables de commande des séquences de fourniture de doses à l'utilisateur ainsi que, avantageusement, des moyens d'enregistrement et de contrôle des séquences de fourniture effective de doses à l'utilisateur.

Selon une caractéristique plus particulière de l'invention, les moyens programmables de commande, ainsi qu'avantageusement les moyens d'enregistrement et de contrôle, comportent un support d'information transportable, avantageusement une carte à puce, connectable au dispositif de commande et, avantageusement, à un poste séparé de programmation et de lecture des informations enregistrées dans ledit support d'information.

Au sens de la présente invention, par doses fournies à un utilisateur, on entend un gaz ou un mélange de gaz sous forme gazeuse, par exemple d'oxygène ou d'air enrichi en oxygène, transportant des particules solides ou liquides d'une substance médicamenteuse.

La présente invention a pour autre objet de proposer un procédé de mise en oeuvre d'un équipement tel que défini ci-dessus, comportant les étapes d'inscrire dans les moyens programmables une durée totale d'une séquence de fourniture de doses ainsi que, avantageusement, au moins une heure journalière de début de séquence de fourniture de doses.

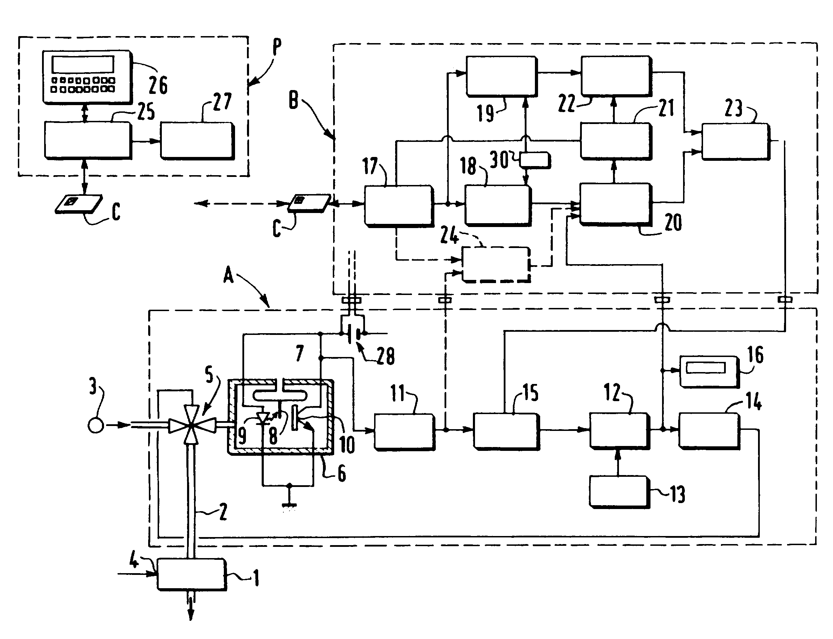

D'autres caractéristiques et avantages de la présente invention ressortiront de la description suivante d'un mode de réalisation, donnée à titre illustratif mais nullement limitatif, faite en relation avec le dessin annexé, sur lequel :
- la figure unique est une représentation schématique d'un équipement de programmation et de surveillance d'administration d'aérosols médicamenteux selon un mode de réalisation de l'invention.

On a représenté sur la figure unique un nébuliseur 1 dans un circuit 2 entre une source de gaz 3 et les voies respiratoires d'un patient, le nébuliseur comprenant une entrée d'air séparée 4. Le circuit 2 comporte, en amont du nébuliseur 1, un dispositif de commande de fourniture de doses de gaz vecteur en provenance de la source 3 comportant une vanne trois voies 5 couplée à un capteur 6 sensible aux phases d'inspiration et d'expiration de l'utilisateur tel que par exemple décrit dans le document EP-A-0.183.593. Il suffira de rappeler ici que le capteur 6 comporte une capsule différentielle 7 portant un volet mobile 8 sélectivement interposable dans le trajet lumineux entre une photodiode 9 et un phototransistor 10 commandant une boucle de commande de l'électrovanne 5 comportant, successivement, un circuit de mise en forme 11, un circuit monostable 12 fournissant un signal de commande d'ouverture de l'électrovanne 5 d'une durée déterminée par un circuit de réglage de durée 13, et un étage de sortie de puissance 14. Typiquement, la durée d'une phase de nébulisation doit être légèrement inférieure à la durée d'une inspiration. En pratique, le circuit de réglage de durée 13 pourra se limiter à un sélecteur sélectionnant trois niveaux de réglage "adulte", "enfant" et "nourrisson".

Selon un aspect de l'invention, entre les circuits 11 et 12 est interposé un circuit de verrouillage sélectif 15, à transistors, à circuits intégrés logiques ou à relais, couplé à un dispositif de contrôle qui va être décrit ci-dessous. De plus, un dispositif de comptage et d'affichage 16 compte et affiche le nombre de signaux d'ouverture de l'électrovanne 5 commandés par le capteur 6.

Dans le mode de réalisation représenté, le dispositif de contrôle comprend un poste 17 de lecture et d'écriture sur un support d'information transportable C, par exemple une bande magnétique multi-pistes ou, de préférence, une carte à puce à mémoire non volatile (SRAM ou "Flash") ou à mémoire programmable. En mode de lecture, le dispositif 17 fournit des signaux à un dispositif 18 de comptage et de lecture d'un nombre de bouffées de gaz délivrées par l'électrovanne 5 au cours d'une séquence de fourniture de gaz et à un dispositif 19 de comptage et de lecture du nombre journalier de séquences de fourniture de doses de gaz, tous deux connectés à une horloge 30. Le dispositif 18 fournit un signal de sortie à un dispositif 20 de détection de fin de séquence couplé en sortie à une mémoire non volatile 21 dont le contenu est susceptible d'être déchargé dans le circuit intégré de la carte C dans le mode écriture du dispositif 17. La mémoire 21 est reliée, ainsi que le dispositif 19, à un dispositif 22 d'autorisation de début de séquence fournissant en sortie un signal adressé, de même qu'un signal de sortie du dispositif 20, à une porte logique 23 commandant le circuit de verrouillage 15. En variante, le dispositif de contrôle peut également comporter un circuit 24 d'analyse de défauts et d'actionnement d'une alarme recevant des signaux en provenance du dispositif 17 et de la sortie du circuit de mise en forme 11 pour fournir un signal de sortie adressé à une entrée du circuit 20 de détection de fin de séquence.

Dans le mode de réalisation représenté, l'équipement est complété par un poste séparé de programmation et de lecture P comportant un dispositif de programmation et de lecture 25 à un microprocesseur auquel est susceptible d'être connectée la carte à puce C, relié à un ensemble clavier de programmation/écran 26 et à une imprimante 27. Avantageusement, la chaîne de commande de l'électrovanne 5 est regroupée dans un module de base indépendant A, pourvu d'une horloge interne (non représentée), le dispositif de contrôle décrit plus haut étant regroupé dans un module de contrôle B susceptible d'être associé au module de base A par des raccords rapides, et prélevant avantageusement, en mode opératoire, son énergie électrique à une source d'alimentation électrique 28 du module de base A.

Le fonctionnement de l'équipement est le suivant :

A un patient déterminé est associée une carte à puce particulière C. Selon le traitement envisagé pour le patient, le médecin, détenteur du poste programmation/lecture P, introduit dans ce dernier la carte C du patient et y inscrit un code d'identification du patient, une heure de prise de médications (début de séquence) et le nombre de bouffées à prendre lors de la prise de la médication (durée de la séquence). La carte est alors remise au patient qui l'introduit dans le dispositif de lecture/écriture 17 de son module de contrôle B qui placera alors le module de base A dans une position d'attente sous contrôle pour ne se mettre en fonctionnement, et donc libérer l'aérosol médicamenteux, qu'à l'heure programmée fixée par le praticien. Ceci revêt un intérêt primordial pour les thérapies chrono-biologiquement dépendantes et pour l'antibiothérapie. De même, le nombre de bouffées libérées à l'inspiration du patient ne pourra dépasser celui pré-programmé par le praticien. Ceci met le praticien et le patient à l'abri d'une erreur par excès, une erreur par défaut pouvant être signalée par l'alarme déclenchée par le circuit 24, dans le cas, par exemple, où les deux conditions suivantes sont réunies : le nombre de bouffées prescrites n'est pas atteint et le temps écoulé depuis la dernière bouffée a dépassé une durée déterminée, typiquement une à deux minutes.

Outre sa fonction de fourniture d'informations pré-programmées, la carte à puce C enregistre en permanence, dans le dispositif 17, le comportement du patient et permet notamment de vérifier des défauts d'observance de la prescription du praticien traduite par la programmation effectuée initialement. Ainsi, lors d'une consultation, le patient rapporte sa carte C au médecin qui, en l'introduisant dans le dispositif 25 du poste P, peut lire, voire imprimer, et vérifier ainsi les dates et heures des séances réellement effectuées ou incomplètement effectuées avec la valeur du défaut d'observance se traduisant en nombre de bouffées déficitaires. En effet, en cas d'échec thérapeutique, la question de l'observance est primordiale : c'est pourquoi la lecture des informations enregistrées sur la carte à puce permet au médecin d'apprécier le respect ou le non-respect de sa prescription.

Quoique la présente invention ait été décrite en relation avec un mode de réalisation particulier, elle ne s'en trouve pas limitée pour autant mais est au contraire susceptible de modifications et de variantes qui apparaîtront à l'homme de l'art. En particulier, l'équipement selon l'invention peut être utilisé pour un grand nombre d'appareils de soins à domicile, notamment la programmation du déclenchement d'une CPAP de ventilation de manière à ne pas être gêné par son fonctionnement à l'endormissement, pour la surveillance de l'observance ainsi que de la qualité de son réglage, ou encore pour la surveillance et le contrôle du nombre d'utilisations d'un concentrateur d'oxygène et la surveillance de la concentration en oxygène libéré.

## Revendications

1. Equipement de fourniture aux voies respiratoires d'un utilisateur de doses d'au moins un gaz contenant des particules d'au moins un produit actif, comprennant, en série dans un circuit de gaz (2) entre une source de gaz (3) et les voies respiratoires, une vanne (5) de distribution de gaz et un nébuliseur (1), la vanne (5) étant pilotée par un dispositif de commande comprenant un capteur (6) sensible aux phases d'inspiration de l'utilisateur et des moyens programmables (C, 17) de commande de séquences de fourniture de doses à l'utilisateur.

2. Equipement selon la revendication 1, ledit dispositif de commande comportant des moyens programmables pour déclencher chaque séquence à des moments prédéterminés.

3. Equipement selon l'une des revendications 1 ou 2, comportant en outre des moyens (13) de réglage de la durée de fourniture de dose durant une séquence.

4. Equipement selon l'une des revendications précédentes, comportant en outre un moyen (16) d'affichage du nombre de doses fournies durant une séquence.

5. Equipement selon l'une des revendications précédentes, comportant des moyens (17, C) d'enregistrement et de contrôle des séquences de fourniture effective de doses.

6. Equipement selon l'une des revendications précédentes, lesdits moyens programmables de commande comportent au moins un support d'information transportable (C) connectable (17) au dispositif de commande.

7. Equipement selon la revendication 6, ledit support d'information transportable (C) formant un support d'enregistrement des moyens d'enregistrement.

8. Equipement selon la revendication 6 ou la revendication 7, ledit support d'informations transportable (C) étant une carte à puce.

9. Equipement selon l'une des revendications 6 à 8, ledit support d'informations transportable (C) étant connectable (25) à un poste (P) de programmation et de lecture d'informations enregistrées dans le support d'informations transportable.

10. Procédé de mise en oeuvre d'un appareil selon l'une des revendications précédentes, comportant l'étape d'inscrire dans les moyens programmables (C) une durée totale de fourniture de doses.

11. Procédé selon la revendication 10, comportant l'étape d'inscrire dans les moyens programmables (C) au moins une heure journalière de début de séquence.

12. Procédé selon l'une des revendications 10 et 11 pour la mise en oeuvre d'un appareil selon la revendication 9, comportant les étapes de connecter le support d'informations (C) au poste (P) de programmation/lecture, d'effectuer les inscriptions dans le support d'informations, de déconnecter le support d'informations du poste de programmation/lecture puis de connecter le support d'informations au dispositif de commande (17).

13. Procédé selon la revendication 12, comportant les étapes de déconnecter le support d'informations (C) du dispositif de commande (17), de connecter le support d'informations au poste (P) de programmation/lecture et de lire les informations enregistrées sur le support d'informations relatives aux séquences passées de fourniture de doses.

## Patentansprüche

1. Einrichtung zur Zuführung von Dosen mindestens eines Gases, das Teilchen mindestens eines Wirkstoffs enthält, in die Atemwege eines Benutzers, mit, in Reihe in einem Gaskreislauf (2) zwischen einer Gasquelle (3) und den Atemwegen, einem Ventil (5) zur Verteilung von Gas und einem Zerstäuber (1), wobei das Ventil (5) durch eine Steuervorrichtung gesteuert wird, die einen auf die Inspirationsphasen des Benutzers reagierenden Sensor (6) und programmierbare Mittel (C, 17) zur Steuerung von Zuführungsfolgen von Dosen an den Benutzer enthält.

2. Einrichtung nach Anspruch 1, wobei die Steuervorrichtung programmierbare Mittel zum Auslösen jeder Folge zu vorbestimmten Zeitpunkten enthält.

3. Einrichtung nach Anspruch 1 oder 2, die des weiteren Mittel (13) zur Einstellung der Dosenzuführungsdauer während einer Folge enthält.

4. Einrichtung nach einem der vorhergehenden Ansprüche, die des weiteren ein Mittel (16) zur Anzeige der Anzahl von während einer Folge zugeführten Dosen enthält.

5. Einrichtung nach einem der vorhergehenden Ansprüche, die Mittel (17, C) zur Aufzeichnung und Kontrolle der Folgen effektiver Dosenzuführung enthält.

6. Einrichtung nach einem der vorhergehenden Ansprüche, wobei die programmierbaren Steuermittel mindestens einen transportierbaren Informationsträger (C) enthalten, der mit der Steuervorrichtung verbunden (17) werden kann.

7. Einrichtung nach Anspruch 6, wobei der transportierbare Informationsträger (C) einen Aufzeichnungsträger der Aufzeichnungsmittel bildet.

8. Einrichtung nach Anspruch 6 oder 7, wobei der transportierbare Informationsträger (C) eine Chipkarte ist.

9. Einrichtung nach einem der Ansprüche 6 bis 8, wobei der transportierbare Informationsträger (C) mit einer Station (P) zum Programmieren und Lesen von im transportierbaren Informationsträger aufgezeichneten Informationen verbunden (25) werden kann.

10. Verfahren zum Einsatz eines Geräts nach einem der vorhergehenden Ansprüche, bei dem man in die programmierbaren Mittel (C) eine Gesamtdauer der Dosenzuführung einschreibt.

11. Verfahren nach Anspruch 10, bei dem man in die programmierbaren Mittel (C) mindestens einen täglichen Zeitpunkt für den Folgebeginn einschreibt.

12. Verfahren nach Anspruch 10 oder 11 zum Einsatz eines Geräts nach Anspruch 9, bei dem man den Informationsträger (C) mit der Programmier-/Lesestation (P) verbindet, um Einschreibungen in den Informationsträger durchzuführen, den Informationsträger von der Programmier-/Lesestation trennt und ihn dann mit der Steuervorrichtung (17) verbindet.

13. Verfahren nach Anspruch 12, bei dem man den Informationsträger (C) von der Steuervorrichtung (17) trennt, ihn mit der Programmier-/Lesestation (P) verbindet und die auf dem Informationsträger aufgezeichneten Informationen über die vergangenen Dosenzuführungsfolgen liest.

## Claims

1. Apparatus for delivering, to the airways of a user, doses of at least one gas containing particles of at least one active product, comprising, in series in a gas circuit (2) between a gas source (3) and the airways, a gas distribution valve (5) and a nebulizer (1), the valve (5) being controlled by a control device comprising a sensor (6) sensitive to the user's inhalation phases and programmable means (C, 17) for controlling sequences of dose delivery to the user.

2. Apparatus according to Claim 1, the said control device including programmable means for initiating each sequence at predetermined moments.

3. Apparatus according to either of Claims 1 and 2, which furthermore includes means (13) for adjusting the duration of dose delivery during a sequence.

4. Apparatus according to one of the preceding claims, which furthermore includes a means (16) for displaying the number of doses delivered during a sequence.

5. Apparatus according to one of the preceding claims, which includes means (17, C) for recording and monitoring the sequences of actual dose delivery.

6. Apparatus according to one of the preceding claims, the said programmable control means including at least one transportable information medium (C) which can be connected (17) to the control device.

7. Apparatus according to Claim 6, the said transportable information medium (C) forming a recording medium for the recording means.

8. Apparatus according to Claim 6 or Claim 7, the said transportable information medium (C) being a smart card.

9. Apparatus according to one of Claims 6 to 8, the said transportable information medium (C) being able to be connected (25) to a station (P) for programming and reading the information recorded in the transportable information medium.

10. Procedure for using an apparatus according to one of the preceding claims, which includes the step of entering a total dose delivery duration into the programmable means (C).

11. Procedure according to Claim 10, which includes the step of entering at least one daily sequence start time into the programmable means (C).

12. Procedure according to either of Claims 10 and 11 for the use of an apparatus according to Claim 9, which includes the steps of connecting the information medium (C) to the programming/reading station (P), of making the entries in the information medium, of disconnecting the information medium from the programming/reading station and then of connecting the information medium to the control device (17).

13. Procedure according to Claim 12, which includes the steps of disconnecting the information medium (C) from the control device (17), of connecting the information medium to the programming/reading station (P) and of reading the information recorded on the information medium relating to past dose delivery sequences.
